# EUROPEAN PATENT APPLICATION

(11) **EP 3 583 968 A1**
(43) Date of publication of application: **25.12.2019**
(21) Application number: 18178527.0
(22) Date of filing: 19.06.2018
(51) Int. Cl.: A61M 11/00, A61M 15/00

(54) **A SYSTEM FOR GENERATING A DROPLET OUTPUT AND A METHOD OF MONITORING CLEANING**

(71) Applicant: Koninklijke Philips N.V., 5656 AE Eindhoven (NL)
(72) Inventor: VON HOLLEN, Dirk, 5656 AE Eindhoven (NL); PRITCHARD, John, 5656 AE Eindhoven (NL)
(74) Representative: de Haan, Poul Erik

(57) **Abstract**

A system for generating a droplet output determines a separation time during which a droplet generator is separated from a main body of the system. This timing information is used to generate advisory information relating to cleaning of the droplet generator. In particular, it is possible to provide a warning when it is detected that a cleaning protocol has not been followed.

## Description

### FIELD OF THE INVENTION

The invention relates to systems for generating droplet outputs, such as nebulizers, and in particular to a method of verifying that a suitable cleaning procedure has been employed.

### BACKGROUND OF THE INVENTION

There are many applications in which generation of droplets, as a spray or mist, is required. These may make use of a droplet generator, which may become clogged over time. One application of particular interest is the delivery of medication in aerosol form.

There are three basic categories of device for delivering a medicament in aerosol form directly to the lungs of a patient during inhalation: metered-dose inhalers (MDI); dry powder inhalers (DPI); and nebulizers.

Generally, medicaments available for use in a MDI are provided in a pressurized formulation, medicaments available for use in a DPI are provided in a dry powder formulation, and medicaments available for use in a nebulizer are provided in an aqueous solution formulation.

This invention is of particular interest to nebulizers, although it is of interest more widely to systems for generating a droplet output. Due to their ease of use, many patients prefer nebulizers for the delivery of aerosolized medicaments. Nebulizers may employ different mechanisms for aerosolizing a medicament. For example, pneumatic (or jet) nebulizers employ compressed air or oxygen to disperse the liquid into aerosol form. An ultrasonic nebulizer, in contrast, employs an electrical source to excite a piezoelectric element which generates high frequency vibrations. These vibrations cause small droplets to separate from the surface of the medicament, thus forming an aerosol. Vibrating mesh technology (VMT) nebulizers employ a mesh having a number of microscopic apertures. A vibration is induced in the mesh (or in another component within the nebulizer) which causes the aqueous-based medicament to be extruded through the mesh apertures. The aqueous-based medicament is ejected from the mesh apertures in aerosol form.

VMT nebulizers have gained in popularity among patients. VMT nebulizers, however, possess some drawbacks. For example, some medicaments delivered by a VMT nebulizer tend to accumulate on the surface of the nebulizer mesh. This accumulation may occlude the microscopic apertures in the mesh, thereby reducing the effectiveness of the nebulizer. For example, total blockage of one or more apertures may decrease the efficiency of the device or ultimately prevent any medicament from being dispensed to the patient. In addition, partial blockage of one or more apertures may negatively affect the particle size or time of delivery, thus possibly reducing or eliminating the beneficial effects of the prescribed therapy.

It is therefore required to clean the mesh regularly of a VMT nebulizer. A typical nebulizer cleaning protocol requires that the nebulizer be disassembled and the mesh removed (either alone or as part of a module). A basic cleaning protocol involves washing mesh with warm soapy water. Afterwards, the mesh is rinsed in tap water and allowed to air dry. After the mesh has dried, it is re-inserted into the nebulizer which thereafter is reassembled for use. Other cleaning protocols have also been suggested for cleaning the mesh by soaking with cleaning agents and drying, or by boiling in water for a certain time followed by drying. Other cleaning protocols for example require the mesh to be placed in a high-temperature dishwasher, in an autoclave, and/or in an ultrasonic cleaner.

These cleaning protocols thus typically all require a time duration of at least several minutes. US 2007/0189919 discloses various cleaning operations for a VMT nebulizer.

The administration of therapy with a typical mesh nebulizer system can be quickly conducted, whereas the subsequent cleaning and drying operations are time consuming as explained above. The user is typically instructed by the manufacturer to clean regularly (i.e. as a minimum to rinse and air dry after use) or for disinfection to follow a set of instructions as discussed above.

There is therefore a risk that a user does not perform the required disassembly, cleaning and reassembly operations, through lack of time or interest.

US 2007/0074722 discloses a nebulizer which provides a reminder that cleaning is needed, if the device is not separated after use. However, this does not enable detection of whether cleaning has actually taken place.

There is therefore a need to assess whether or not correct cleaning has taken place, so that correct operation of the nebulizer can be maintained.

### SUMMARY OF THE INVENTION

The invention is defined by the claims.

According to examples in accordance with an aspect of the invention, there is provided a system for generating a droplet output, comprising:
a main body;
a droplet generator, wherein the droplet generator is separable from the main body for cleaning;
a sensor for detecting separation of the droplet generator from the main body;
a timer; and
a controller, which is adapted to:
   control the timer to determine a separation time during which the droplet generator is separated from the main body; and
   generate advisory information for output to a user of the system relating to cleaning of the droplet generator, and which information takes into account the determined separation time.

This system detects when the droplet generator is separated for cleaning, and also monitors a time duration of that separation. The separation time must be more than a certain threshold in order for the recommended cleaning protocol to have been followed, so the device is able to provide a warning when it is determined that the correct cleaning protocol cannot have been followed.

The controller may be adapted to set a threshold time period, and is adapted to generate advisory information to indicate that correct cleaning has not been conducted if the determined separation time is less than the threshold time period.

The threshold time period is for example longer than the time that would be required to perform a cursory rinse, but shorter than the typical time that would be required to perform the full recommended cleaning protocol. Thus, it enables discrimination between a cursory rinse and a full wash.

The threshold time period is for example at least 5 minutes, for example at least 10 minutes.

The controller is preferably adapted to determine a separation time during which the droplet generator is separated from the main body either since the last use of the system or after a set number of uses of the system.

The device will thus be set to the recommended cleaning protocol. This may be a rinse after each use, but a full clean after a fixed number of uses, or it may be a full clean after each use. Thus, generally, the device preferably monitors when the recommended cleaning protocol is required based on usage information as well as the recommended cleaning schedule.

The device may also monitor a time since the last detected full clean, so that regardless of use, a clean is recommended after a fixed time period.

The system may further comprise a timer system for timing a dose delivery time period, and wherein the controller is adapted to generate the advisory information further taking into account the dose delivery time period.

The time taken for a dose delivery depends on the state of cleanliness. Thus, after a cleaning operation, it is expected that there is a step change in the dose delivery time period. This provides a way to determine that actual cleaning has taken place, in addition to detecting device separation.

The timing system for example comprises a dry detection system for detecting an end of dose, and a timer. By timing until a dry state is detected, the dose delivery time is obtained.

The system may comprise a driver for providing a drive signal to the droplet generator, wherein the dry detection system comprises a system for monitoring characteristics of the drive signal or electrical characteristics of the droplet generator. The drive signal may be a drive current. This provides one way to implement dry detection.

A liquid level sensor may be provided for determining when a liquid sample has been converted into droplets. Thus, the timing may be carried out from the start of a droplet delivery period to the point when the liquid level reaches a fixed point (which may or may not be delivery of the full sample). Liquid level detection provides another way to implement dry detection.

In all cases, when the droplet generator is partially clogged, the generation of a droplet output (e.g. for atomization of a fixed dose of medication) takes longer. Thus, the additional monitoring of the operation time provides a further indication of the state of cleanliness of the droplet generator, and this additional information may be used to make the cleaning advice more accurate.

The system may comprise a vibrating mesh nebulizer and the droplet generator comprises a nebulizer mesh. The liquid sample is then a dose of a medicament.

In this case, the nebulizer mesh may be part of a mouthpiece, and the sensor is for detecting separation of the mouthpiece from the main body. The mouthpiece may be subjected to the cleaning protocol as a whole, or it may require further disassembly.

The invention also provides a method for monitoring cleaning of a system for generating a droplet output which comprises a droplet generator and a main body which are separable for cleaning of the droplet generator, the method being implemented by the system, and comprising:
detecting separation of the droplet generator from the main body;
determining a separation time during which the droplet generator is separated from the main body; and
generating and outputting advisory information to a user of the system relating to cleaning of the droplet generator, which information takes into account the determined separation time.

The method for example comprises setting a threshold time period, and generating advisory information to indicate that correct cleaning has not been conducted if the determined separation time is less than the threshold time period.

The method may also comprise determining an operation time for generating the droplet output and generating the advisory information further takes into account the determined operation time.

The operation time may be obtained based on detecting when the droplet generator becomes dry thereby signaling the end of a dose delivery, and timing to this point in time.

The system preferably comprises a vibrating mesh nebulizer and the droplet generator comprises a nebulizer mesh. The mesh is preferably part of a mouthpiece, and detecting separation of the mesh from the main body comprises detecting separation of the mouthpiece from the main body.

The invention may be implemented at least in part in software, and the invention thus also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known vibrating mesh nebulizer;
Figure 2 shows the nebulizer of Figure 1 as an exploded view;
Figure 3 shows in schematic form a nebulizer in accordance with an example of the invention; and
Figure 4 shows a cleaning monitoring method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a droplet generating system which determines a separation time during which a droplet generator is separated from a main body of the system. This timing information is used to generate advisory information relating to cleaning of the droplet generator. In particular, it is possible to provide a warning when it is detected that a cleaning protocol has not been followed.

The invention is of particular interest for vibrating mesh nebulizers. The general construction and operation of a vibrating mesh nebulizer will first be described, before the modification in accordance with the invention is explained.

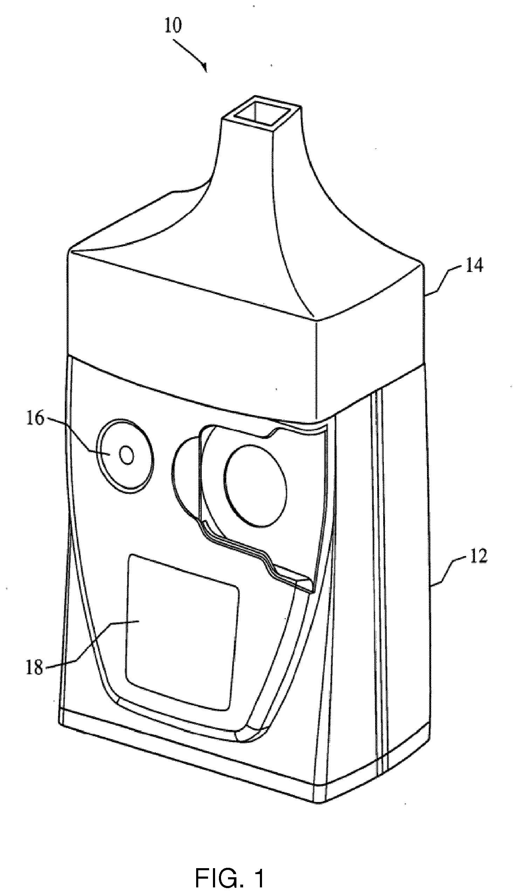

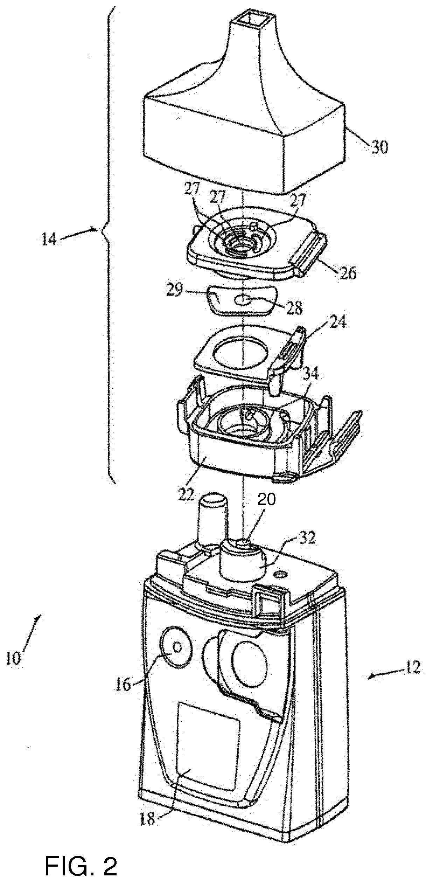

Figure 1 is an isometric view and Figure 2 is an exploded view, of a VMT nebulizer 10. These images are taken from US 2007/0189919.

The nebulizer 10 includes a lower portion in the form of a main body 12 and an upper portion in the form of a mouthpiece 14. The main body 12 includes a power supply source (not shown) such as a rechargeable battery pack, an on/off switch 16, a display 18, and a horn device 20. The main body 12 houses circuitry (not shown) used to control the operation of the nebulizer 10. In the exemplary embodiment, the circuitry includes a microprocessor which is structured to access instructions and control data stored, for example, on a disc and/or a memory. The instructions may include the dosage amount, the dosing frequency, and the number of doses that may be delivered. The control data may include the medicament lot number and expiration date. The microprocessor is adapted to execute the routines in response to the instructions and control data and, in conjunction with the other circuitry, cause the nebulizer 10 to dispense the proper dose of medicament to a patient.

The mouthpiece 14 is removably connected to the main body 12, and includes a medicament chamber 22, a guide 24, a flexible housing 29, a chamber lid 26, and a mouthpiece housing 30. The horn device 20 extends from a boss 32 which fits into opening 34 of the chamber 22. The chamber 22 is structured to hold an aqueous-based medicament therein. The chamber 22 along with guide 24 directs the medicament towards horn device 20. The flexible housing 29 holds a mesh 28 having an upper surface and a lower surface.

The horn device 20 includes a piezoelectric element (not shown) that is in electrical communication with the circuitry (not shown) contained within the main body 12. The horn 20 and piezoelectric element of the main body 12 vibrates the mesh 28 of to form a nebulization mechanism. More specifically, the lower surface of the mesh 28 contacts a top surface of the horn 20. The piezoelectric element is structured to oscillate when driven by the circuitry contained within the main body 12. A vibratory motion caused by the oscillation of the piezoelectric element is transferred to the horn 20 and to the mesh 28. The vibratory motion causes the aqueous-based medicament within chamber 22 to pass through a number of apertures of the mesh 28. The medicament leaving the apertures at the upper surface of the mesh 28 is aerosolized.

After exiting the apertures of the mesh, the aerosolized medicament passes from the upper surface of mesh 28 through openings 27 toward the exit of the mouthpiece 14. The aerosolized medicament can then be inhaled by a patient.

The mouthpiece 14 of the nebulizer 10 can be disassembled into its component parts, for example, during cleaning. Particularly, the chamber lid 26 and mesh 28 may be separated from the other components of the nebulizer 10. The mesh 28 may remain housed within the chamber lid 26 during cleaning and/or the mesh 28 maybe removed from the chamber lid 26 and cleaned separately.

This is just one example of possible nebulizer architecture, and others are possible.

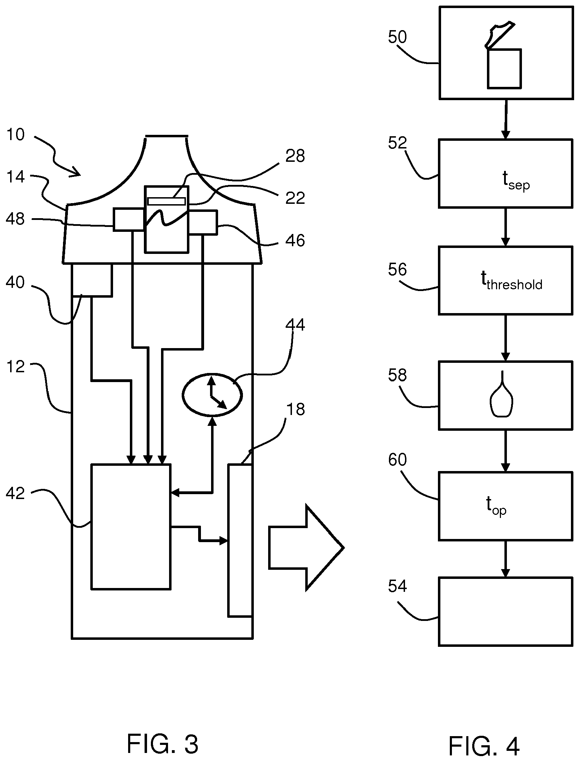

Figure 3 shows a vibrating mesh nebulizer in schematic form, to illustrate the additional features to the known system of Figures 1 and 2.

The nebulizer 10 again comprises a main body 12 and a mouthpiece 14 which includes a nebulizer mesh 28. The nebulizer mesh 28 is separable from the main body 12 for cleaning.

A sensor 40 is provided for detecting separation of the nebulizer mesh from the main body 12, in particular by detecting separation of the mouthpiece 14 from the main body 12.

The sensor may comprise a contact sensor, whereby electrical contact is made when the mouthpiece and main body are connected. Other sensors may be used, such an optical sensor (e.g. an LED and a photodiode for detecting reflection from the mouthpiece when it is connected) or any other contact or proximity sensor.

The main body has a controller 42 which receives the information from the sensor 40. The controller also includes a drive signal generator for generating a drive signal for the mesh 48. This drive signal for example has a frequency chosen to match a resonant frequency of the mesh which is typically of the order of magnitude of 100kHz such as 128kHz. A drive current is provided to the mesh 28 to set it into resonance.

A timer 44 is used for determining time periods, under the control of the controller 42. The timer 44 is operated to measure a separation time during which the nebulizer mesh, i.e. the mouthpiece, is separated from the main body. Advisory information is then generated by the controller for output to a user of the nebulizer using the display 18 (and/or an audible output). This output provides information relating to cleaning of the nebulizer mesh. In particular, it provides a warning that correct cleaning is overdue and/or an instruction to perform the required cleaning task. This warning or instruction takes into account the determined separation time.

The separation time needs to be more than a certain threshold in order for the recommended cleaning protocol to have been followed, so the device is able to provide a warning when it is determined that the correct cleaning protocol cannot have been followed.

The threshold is longer than the time that would be required to perform a cursory rinse but shorter than the typical time that would be required to perform the full recommended cleaning protocol. Thus, it enables discrimination between a cursory rinse and a full wash. The threshold time period is for example at least 5 minutes, for example at least 10 minutes. It will be set to a value which depends on the recommended cleaning procedure, since that procedure will require a minimum time period to be completed correctly.

The cleaning protocol being monitored may be required after each use of the nebulizer, so the monitoring takes place after each use. However, the cleaning protocol may instead require a rinse after each use and a complete clean after a set number of uses or after a set time such as weekly. Thus, the threshold may be applied either each time the mesh is separated from the main body or only after a set number of uses of the nebulizer or set period of time.

There may be multiple thresholds. For example it may be required that there is separation after each use to ensure that at least a rinse has been carried out. This implies a short time threshold, e.g. 1 minute. A longer time threshold may then be applied when a full wash has been required, e.g. 10 minutes.

Different cleaning protocols are possible, for example depending on the condition being managed/treated. Thus, the monitoring of the cleaning takes account of the specified cleaning protocol.

To the extent described above, the system is able to detect failure to have conducted the required cleaning procedure. Additional sensing may be used to provide greater confidence that the required cleaning has been carried out.

A first effect of cleaning is that the dose delivery time changes. Thus, Figure 3 shows a timing system 44, 46 for measuring the dose delivery time and the controller 42 is then adapted to generate the advisory information further taking into account the dose delivery time, which provides a positive indication that cleaning has actually taken place.

The timing system 44, 46 for example measures a time from when the dose begins to when the mesh has become dry, and thus includes a dry detection system 46. detecting an end of dose. The timer 44 described above may form part of the timing system 44, 46. In one example, the dry detection system 46 can be implemented by monitoring a drive current to the mesh 28. The mesh drive signal brings the mesh into resonance, and the load presented by the mesh influences the required drive current. This load depends on the wet or dry state of the mesh, so that a change (drop) in current is observed when the mesh is dry, at the end of the dose delivery.

In this way, changes in the operation time for nebulizing a dose of drug after the device has been reconnected will reflect that the mesh has indeed been cleaned. It is known that the operation time is dependent on the state of cleanliness of the mesh, and indeed it is known to require a user to measure this time to verify the correct functioning of the nebulizer.

The monitoring of the drive current to distinguish between wet and dry states is discussed in more detail in WO 2015/010809.

The dose delivery time after a full cleaning event should return to a baseline value. Thus, the dose delivery time may be used not only to confirm that cleaning has taken place, but also to provide an indication of the effectiveness of the cleaning.

Thus, an additional alert may be provided to indicate that, even though cleaning seems to have taken place (based on the separation time), the cleaning has not been effective (based on the dose time not returning to the expected baseline value or with a threshold difference). This may either indicate the end of life of the mesh or it may indicate that the correct cleaning protocol was not followed.

The current monitoring provides one way to detect a dry mesh at the end of the delivered dose, and thereby enable the dose delivery time to be measured. An alternative approach is to monitor the quantity of liquid, and a liquid level sensor 48 may be provided for determining when the dose, or a fixed portion of the dose, of drug to be delivered to the user has been atomized. Thus, the timing may be carried out from the start of a dose delivery to the point when the liquid level reaches a fixed point (which may or may not be delivery of the full dose) or the timing may be carried out from the start of a dose delivery and a detection of a dry state based on monitoring the drive current to the mesh (or monitoring other electrical characteristics, such as measuring an impedance).

A liquid level sensor for example may be implemented as liquid sensing electrodes at a certain location within the dose chamber which detect the presence or absence of liquid based on a change in capacitance or resistance. Any suitable liquid sensing or level sensing approach may be used.

Figure 5 shows a method for monitoring cleaning of a system for generating a droplet output. The method comprises:
in step 50, detecting separation of a droplet generator from a main body;
in step 52 determining a separation time (tₛₑₚ) during which the droplet generator is separated from the main body; and
in step 54 generating and outputting advisory information to a user of the system relating to cleaning of the droplet generator, which information takes into account the determined separation time.

The method for example also comprises in step 56 setting a threshold time (t_{threshold}) period and in step 58 detecting when the droplet generator has become dry at the end of a dose delivery.

The method may also comprise in step 60 determining an operation time (tₒₚ) for generating the droplet output and generating the advisory information further takes into account the determined operation time.

As is clear from the description above, the method is preferably implemented by a vibrating mesh nebulizer, and the operation time is the time to atomize a fixed dose (or proportion thereof) of a liquid medicament.

The invention may be applied to other droplet generating systems which have a cleaning protocol for the droplet generator. Examples are delivery systems for pesticides, disinfection, painting, scent delivery or spray cooling systems.

As discussed above, embodiments make use of a controller. The controller can be implemented in numerous ways, with software and/or hardware, to perform the various functions required. A processor is one example of a controller which employs one or more microprocessors that maybe programmed using software (e.g., microcode) to perform the required functions. A controller may however be implemented with or without employing a processor, and also maybe implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media maybe fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system for generating a droplet output, comprising:
a main body (12);
a droplet generator (28), wherein the droplet generator is separable from the main body for cleaning;
a sensor (40) for detecting separation of the droplet generator from the main body;
a timer (44); and
a controller (42), which is adapted to:
control the timer (44) to determine a separation time during which the droplet generator is separated from the main body; and
generate advisory information for output to a user of the system relating to cleaning of the droplet generator, and which information takes into account the determined separation time.

2. A system as claimed in claim 1, wherein the controller (42) is adapted to set a threshold time period, and is adapted to generate advisory information to indicate that correct cleaning has not been conducted if the determined separation time is less than the threshold time period.

3. A system as claimed in claim 2, wherein the threshold time period is at least 5 minutes, for example at least 10 minutes.

4. A system as claimed in any one of claims 1 to 3, wherein the controller is (42) adapted to determine a separation time during which the droplet generator is separated from the main body since:
the last use of the system; or
a set number of uses of the system.

5. A system as claimed in any one of claims 1 to 4, further comprising a timer system (44, 46) for timing a dose delivery time period, and wherein the controller is adapted to generate the advisory information further taking into account the dose delivery time period.

6. A system as claimed in claim 5, wherein the timing system (44, 46) comprises a dry detection system (46) for detecting an end of dose and a timer (44).

7. A system as claimed in claim 6, comprising a driver for providing a drive signal to the droplet generator, wherein the dry detection system (46) comprises a system for monitoring characteristics of the drive signal or electrical characteristics of the droplet generator.

8. A system as claimed in any one of claims 1 to 7, wherein the system comprises a vibrating mesh nebulizer and the droplet generator (28) comprises a nebulizer mesh.

9. A system as claimed in claim 8, wherein the nebulizer mesh (28) is part of a mouthpiece (14), wherein the sensor is for detecting separation of the mouthpiece from the main body.

10. A method for monitoring cleaning of a system for generating a droplet output which comprises a droplet generator (28) and a main body (12) which are separable for cleaning of the droplet generator, the method being implemented by the system and comprising:
(50) detecting separation of the droplet generator from the main body;
(52) determining a separation time during which the droplet generator is separated from the main body; and
(54) generating and outputting advisory information to a user of the system relating to cleaning of the droplet generator, which information takes into account the determined separation time.

11. A method as claimed in claim 10, comprising (56) setting a threshold time period, and generating advisory information to indicate that correct cleaning has not been conducted if the determined separation time is less than the threshold time period.

12. A method as claimed in claim 11, further comprising (60) determining an operation time for generating the droplet output and generating the advisory information further takes into account the determined operation time.

13. A method as claimed in claim 12, comprising (58) detecting when the droplet generator becomes dry thereby signaling the end of a dose delivery to enable the operation time to be determined.

14. A method as claimed in any one of claims 10 to 13, wherein the system comprises a vibrating mesh nebulizer and the droplet generator comprises a nebulizer mesh (28).

15. A computer program comprising computer program code means which is adapted, when said program is run on a computer, to implement the method of any one of claims 10 to 14.
